# EUROPEAN PATENT SPECIFICATION

(11) **EP 1 862 125 B1**
(45) Date of publication and mention of the grant of the patent: **03.08.2011**
(21) Application number: 07010707.3
(22) Date of filing: 30.05.2007
(51) Int. Cl.: A61B 17/04, A61B 17/06, A61B 17/28, A61B 17/30

(54) **Medical suturing tool with gripping device**
Medizinisches Nahtinstrument mit Greifvorrichtung
Outil de suture chirurgicale avec dispositif de préhension

(30) Priority: 31.05.2006 JP 2006151429; 05.06.2006 JP 2006156073
(43) Date of publication of application: 05.12.2007
(73) Proprietor: Covidien AG, 8212 Neuhausen am Rheinfall (CH)
(72) Inventor: Harada, Hisataka, Fukuroi-shi, Shizuoka-ken (JP); Mikami, Hidetomo, Fukuroi-shi, Shizuoka-ken (JP); Suzuki, Mihoko, Fukuroi-shi, Shizuoka-ken (JP)
(74) Representative: Tomlinson, Edward James

(56) References cited:
- EP-A- 1 598 017
- WO-A-95/22932
- WO-A-96/20649
- WO-A-97/35523
- WO-A-99/34744
- WO-A-2005/094697
- US-A- 5 281 237
- US-A- 5 364 410
- US-A- 5 722 981

## Description

### Technical Field

The present invention relates to a medical suturing tool for stitching a part to be sutured inside a patient's body.

### Technical Background

It is conventional practice to use a medical suturing tool in order to fixedly stitch an area to be sutured inside the body of a patient, and especially an area to be sutured consisting of skin and internal organs. For example, liquid food and drink such as liquid food diets and nutrients are supplied inside the stomach of people who have reduced ability to ingest food orally under their own power due to old age or illness, using a gastrostomy tube, but this gastrostomy tube is attached to the region of the patient's abdomen by the formation of a hole. In such cases, the abdominal wall and the gastric wall are fixed beforehand using a medical suturing tool in order to attach the gastrostomy tube properly (see Japanese Unexamined Patent Application Publication H5-161655, for example).

These medical suturing tools are provided with two puncture needles which are arranged in parallel with a space maintained between them, and when suturing is performed these two puncture needles are first of all simultaneously pierced into the area to be sutured of the patient. Next, suturing thread is passed through one of the puncture needles, and also an inner needle to which a loop made of wire is joined at its tip end part is passed through the other puncture needle, and the inner needle is removed from the puncture needle in a state in which the suturing thread is gripped by the loop inside the patient's body. Then, after the two puncture needles have been removed from the patient, both side portions of the suturing thread which are protruding outside the patient's body are joined, whereby the suture is complete. Furthermore, the tip end part of the puncture needle in which the inner needle is inserted is formed with a curved part and its tip end opening is oriented horizontally, and because of this, when the inner needle is pushed inside the puncture needle, the loop extends in the horizontal direction and protrudes to the outside, and therefore the suturing thread is gripped.

Further suturing devices are known from the documents US 5,722,981, WO 96/20649, US 5,281,237, WO 99/34744, EP 1 598 017A and WO 2005/094697. US 5,722,981 describes an arrangement in which a single suture insertion needle and a single suture retrieval needle are arranged. A handle arrangement which acts on a retractor rod is attached to a frame by two spring elements. EP 1 598 017A describes suturing arrangements including one having a single suture retrieval needle and two suture insertion needles.

### Summary of the Invention

The present invention provides a medical suturing tool according to claim 1. Preferred aspects of the invention are provided according to the dependent claims.
However, with conventional medical suturing tools, when the two puncture needles are pulled out from the patient, the two puncture needles must be handled together with the suturing thread while it is still being gripped by the loop, and therefore there are problems in that this handling is difficult and also the stability of the suturing is adversely affected.

The present invention is a device to deal with these kinds of problems, and it aims to provide a medical suturing tool which is easily handled and which offers improved stability of suturing.

In order to achieve the aim described above the features of the configuration of the medical suturing tool pertaining to the present invention lie in the fact that it is a medical suturing tool in which a linear gripping member is inserted from the base end part side of an insertion hole in a retrieval puncture needle, and a gripping part at the tip end of the linear gripping member is made to protrude from an opening part which is formed at the tip end of the retrieval puncture needle; also, a suturing thread is inserted from the base end part side of an insertion hole in an insertion puncture needle which is arranged approximately parallel and held with a prescribed space between it and the retrieval puncture needle, and, in a state in which the tip end part of the suturing thread is made to protrude from the opening part formed at the tip end of the insertion puncture needle to extend to the gripping part side of the linear gripping member, the suturing thread can be gripped by the gripping part by causing the gripping part of the linear gripping member to retreat to the base end part side of the retrieval puncture needle; in said medical suturing tool a retracting operating member is linked to the base end side of the linear gripping member, the retracting operating member is retractably attached to the base end part side of the retrieval puncture needle, and also provision is made for advancement prevention means for preventing the advance of the retracting operating member with respect to the retrieval puncture needle when the retracting operating member is made to retreat, causing the gripping part of the linear gripping member to retreat to the base end part side of the retrieval puncture needle.

The medical suturing tool pertaining to the present invention configured in the manner described above is provided with the retracting operating member which is joined to the base end side of the linear gripping member, and the advancement prevention means for preventing the advance of the retracting operating member from a retreated position. Then, the gripping part of the linear gripping member can retract from the opening which is formed at the tip end of the retrieval puncture needle due to the fact that the retracting operating member is made to retract. Furthermore, when the retracting operating member is made to retreat, causing the retreat of the gripping part of the linear gripping member to the base end part side inside the retrieval puncture needle, the retracting operating member cannot advance with respect to the retrieval puncture needle due to the advancement prevention means.

In other words, the time when said retracting operating member is made to retreat, causing the retreat of the gripping part of the linear gripping member to the base end part side inside the retrieval puncture needle constitutes the time when the suturing thread is gripped by the gripping part or when the suturing thread is gripped by the gripping part and pulled inside the retrieval puncture needle, and it is therefore possible to prevent the release of the grip (engagement) of the suturing thread by virtue of the gripping part due to the fact that the advance of the retracting operating member towards the inside of the retrieval puncture needle is prevented. By virtue of this, it is possible to pull the retrieval puncture needle and the insertion puncture needle out from the patient's body without in particular manually handling the linear gripping member or the suturing thread. Furthermore, the engagement of the gripping part of the linear gripping member and the suturing thread is not readily released, and therefore the stability of the suturing operation is improved.

Moreover, the advance of the retracting operating member in this case involves the movement of the linear gripping member to inside the patient's body in a state in which the medical suturing tool has pierced the patient's body, and the retreat of the retracting operating member involves the movement of the linear gripping member to outside the patient's body. Furthermore, the advancement prevention means are configured by urging means for urging the retracting operating member to the side of retreat, and engagement means or the like provided with release means, and when the retracting operating member is moved to the side of advance, forwards movement is possible by means of a prescribed method and according to the intentions of the operator.

Another feature of the configuration of the medical suturing tool pertaining to the present invention lies in the fact that the advancement prevention means are configured by an elastic member which urges the retracting operating member towards the outer part of the base end side of the retrieval puncture needle. By virtue of this it is possible to configure the advancement prevention means with a simple member. Furthermore, the operator sets the degree of elasticity of the elastic member in this case so that it is possible to move the retracting operating member forwards in resistance to the elasticity of the elastic member, and, moreover, so that it is possible to prevent the retracting operating member from advancing due to the pull of the suturing thread when the retrieval puncture needle and the insertion puncture needle are pulled out from the patient's body. Furthermore, it is possible to use a coil spring or cord-shaped rubber etc. for the elastic member in this case.

Another feature of the configuration of the medical suturing tool pertaining to the present invention lies in the fact that the advancement prevention means are configured by a retreat side fixing member which fixes the retracting operating member. It is possible to use as the retreat side fixing member in this case a member with which the retracting operating member can be fixed by means of the engagement between an engagement part and a part to be engaged, and furthermore with which the retracting operating member is able to move by means of the release of the engagement between said engagement part and part to be engaged. By virtue of this, it is possible to more reliably prevent the movement of the retracting operating member to the side of advance.

Another feature of the configuration of the medical suturing tool pertaining to the present invention lies in the fact that provision is made for advancement side fixing means for fixing the retracting operating member with respect to the retrieval puncture needle when the retracting operating member is made to advance, causing the gripping part of the linear gripping member to protrude from the opening part in the retrieval puncture needle. By virtue of this it is possible to fix the position of the gripping part when the gripping part of the linear gripping member is protruding from the opening part of the retrieval puncture needle, and therefore this simplifies handling when the tip end part of the suturing thread is protruding from the opening part which is formed at the tip end of the insertion puncture needle and engaging with the gripping part of the linear gripping member. In other words, there is no need to manually hold the retracting operating member back when the suturing thread is inserted inside the insertion puncture needle.

### Brief Description of the Figures

Embodiments of the present invention will now be described, by way of example only, with reference to the accompanying drawings in which:
Figure 1 is an oblique view showing the medical suturing tool pertaining to a first embodiment of the present invention;
Figure 2 is an oblique view showing a state in which the suturing thread is engaged with the linear gripping member of the medical suturing tool shown in Figure 1;
Figure 3 is a front view showing a state in which the expandable engagement part is being pushed downwards close to the gripping part;
Figure 4 is a front view showing a state in which the expandable engagement part is pushed inside the gripping part;
Figure 5 is a front view showing a state in which the expandable engagement part is inside the gripping part and the end part is engaged with the engagement hole;
Figure 6 is a cross-sectional view showing a state in which the medical suturing tool has pierced the abdomen part;
Figure 7 is a cross-sectional view showing a state in which the gripping part of the linear gripping member is protruding from the opening part of the retrieval puncture needle;
Figure 8 is a cross-sectional view showing a state in which the suturing thread is inside the gripping part of the linear gripping member;
Figure 9 is a cross-sectional view showing a state in which the suturing thread is engaged with the gripping part;
Figure 10 is a cross-sectional view showing the suturing thread after the medical suturing tool has been pulled out from the abdomen part;
Figure 11 is a cross-sectional view showing a state in which the suture is complete;
Figure 12 is an oblique view showing the medical suturing tool pertaining to a second embodiment of the present invention;
Figure 13 is an oblique view showing a state in which the suturing thread is engaged with the linear gripping member of the medical suturing tool shown in Figure 12;
Figure 14 is a front view showing the sliding engagement member and the linear gripping member of the medical suturing tool in a state in which the gripping part is protruding from the retrieval puncture needle and is extended;
Figure 15 is a front view showing the sliding engagement member and the linear gripping member of the medical suturing tool in a state in which the gripping part has retreated inside the retrieval puncture needle and is extending in a straight line;
Figure 16 is a cross-sectional view showing a state in which the medical suturing device pertaining to the second mode of embodiment has pierced the abdomen part;
Figure 17 is a cross-sectional view showing a state in which the gripping part of the medical suturing tool pertaining to the second mode of embodiment is protruding from the opening part of the retrieval puncture needle;
Figure 18 is a cross-sectional view showing a state in which the suturing thread is inside the gripping part of the medical suturing tool pertaining to the second mode of embodiment;
Figure 19 is a cross-sectional view showing a state in which the suturing thread in the medical suturing tool pertaining to the second mode of embodiment is engaged with the gripping part;
Figures 20 and 21 are representations of the gripping member of the first and second embodiments;
Figures 22 and 23 illustrate the gripping action of the gripping member of Figures 20 and 21;
Figure 24 illustrates a third embodiment of the invention;
Figures 25-31 illustrate the operation of the third embodiment of the invention;
Figure 32 and 33 are illustrations of a suture inserted into a patient using the third embodiment; and
Figures 34-37 illustrate a fourth embodiment of the invention and its operation.

### Detailed Description of the Invention

### First Embodiment

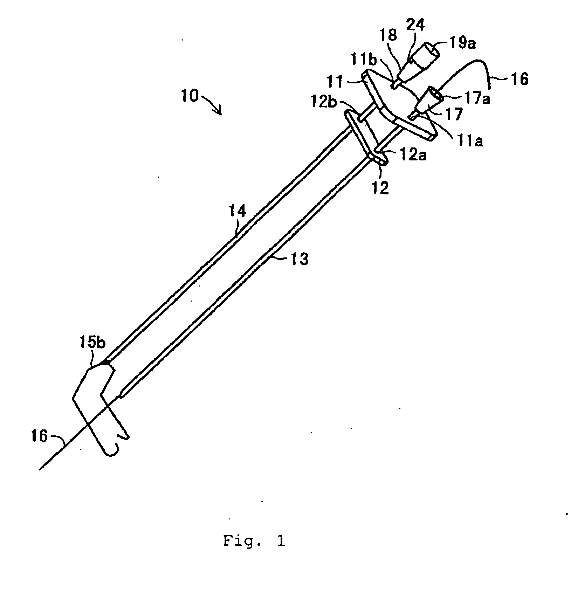

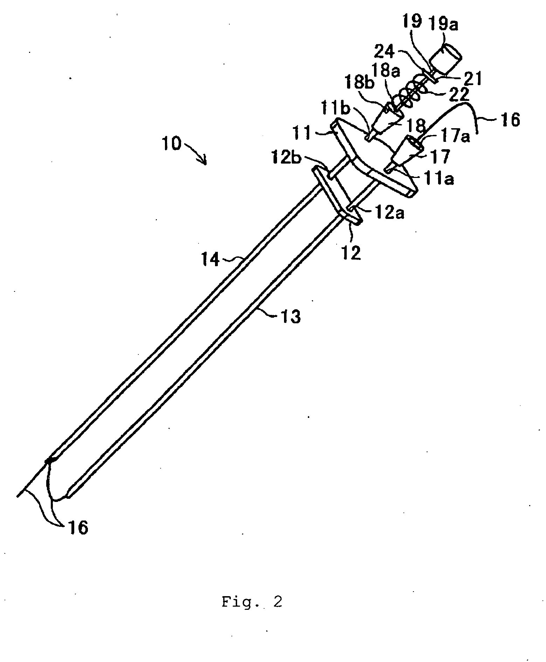

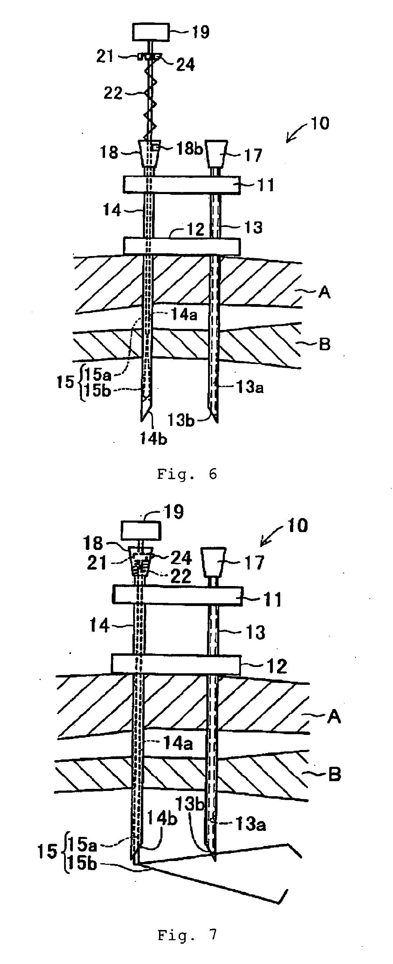

The first mode of embodiment of the present invention will be described below using the figures. Figure 1 and Figure 2 show a medical suturing tool 10 pertaining to the same mode of embodiment. Said medical suturing tool 10 is configured from an upper part holding tool 11 and a lower part holding tool 12, a pair of puncture needles comprising an insertion puncture needle 13 and retrieval puncture needle 14 which are detachably mounted on said upper part holding tool 11 and lower part holding tool 12, a linear gripping member 15 (see Figures 6 to 9), and a suturing thread 16.

The upper part holding tool 11 and the lower part holding tool 12 respectively comprise a moulded body made of a resin material, the upper part holding tool 11 being formed as an approximately square plate shape which is formed with cut-out angled parts having a curved surface, and the lower part holding tool 12 being formed as an approximately rectangular plate shape which is formed with cut-out angled parts having a curved surface. Then, circular holding holes 11a, 11b are formed in both side portions of the upper part holding tool 11 so as to be a prescribed distance apart with the centre point of the upper part holding tool 11 lying between them, and circular holding holes 12a, 12b are formed in both side portions along the longitudinal direction of the lower part holding tool 12 with the same space between them as that between the holding holes 11a, 11b.

The insertion puncture needle 13 is configured by a stainless steel cylindrical body inside which an insertion hole 13a (see Figures 6 to 9) is formed, and a gripping part 17 made of resin is attached to its base end part (upper end part). The upper part of said gripping part 17 is formed as a cylindrical shape with a large diameter while the lower part is formed as a cylindrical shape with a small diameter, and a guide hole 17a which links in communication with the insertion hole 13a is formed inside it. The upper part of said guide hole 17a is formed with a large diameter while the lower part is formed with a small diameter so as to follow the outer peripheral surface of the gripping part 17, and by virtue of this the suturing thread 16 is easily inserted inside the insertion hole 13a of the insertion puncture needle 13 from the top of the gripping part 17.

Furthermore, the tip end part (lower end part) of the insertion puncture needle 13 has an oblique section, and is formed with an opening part 13b which is visible from the lateral direction (the retrieval puncture needle 14 side). Then, said insertion puncture needle 13 is inserted and held in the holding hole 11a of the upper part holding tool 11 and the holding hole 12a of the lower part holding tool 12 in a state in which the opening part 13b is oriented in the direction of the central part of the upper part holding tool 11 and the lower part holding tool 12. The upper part holding tool 11 holds the portion in the vicinity of the base end part of the insertion puncture needle 13, and the lower part holding tool 12 holds the portion which is somewhat further towards the lower side than the base end part of the insertion puncture needle 13, with a gap maintained between said tool and the upper part holding tool 11. The attachment position of the lower part holding tool 12 in this case with respect to the insertion puncture needle 13 is appropriately set in accordance with the degree of protrusion of the portion of the insertion puncture needle 13 further to the lower side than the lower part holding tool 12.

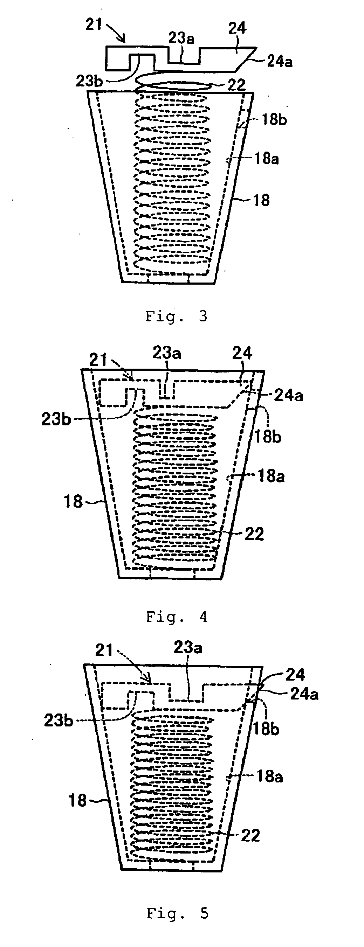

The retrieval puncture needle 14 is configured by a cylindrical body made of stainless steel inside which an insertion hole 14a is formed in the same way as for the insertion puncture needle 13, and a gripping part 18 made of resin is attached to the base end part. The upper part of said gripping part 18 is formed as a columnar shape with a large diameter while the lower part is formed as a columnar shape with a small diameter, and a penetration hole 18a which links in communication with the central part of the insertion hole 14a which runs in the axial direction thereof is formed to pass through vertically. Then, an engagement hole 18b which passes from the inside to the outside is provided at the upper part portion on the peripheral surface of the gripping part 18, as shown in Figures 3 to 5. Furthermore, the tip end part of the retrieval puncture needle 14 has an oblique section, and is formed with an opening part 14b which is visible from the lateral direction (the insertion puncture needle 13 side).

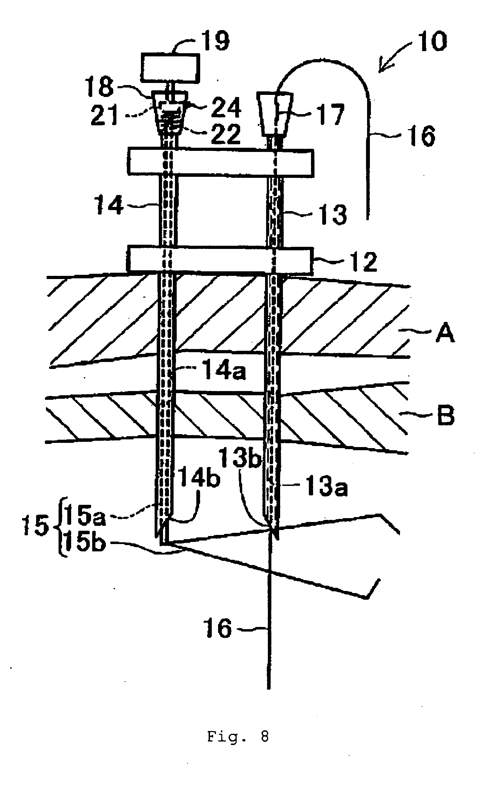

As shown in Figures 6 to 9, the linear gripping member 15 is configured by a linear insertion rod 15a which is configured by a narrow rod made of stainless steel, and a gripping part 15b comprising a fine wire extending from the tip end part of the insertion rod 15a and forking, on which wire a bent part is formed at the respective tip end parts. The linear gripping member 15 can be inserted inside the insertion hole 14a of the retrieval puncture needle 14, and the gripping part 15b extends from the tip end part of the insertion rod 15a so as to spread with a gap between the tip end parts in the direction orthogonal to the insertion rod 15a. Furthermore, the gripping part 15b is deformable, and when it is positioned inside the insertion hole 14a of the retrieval puncture needle 14 it extends to become approximately linear, as shown in Figures 6 to 9, and when it is protruding from the opening part 14b of the retrieval puncture needle 14, it extends orthogonally from the insertion rod 15a to spread to the insertion puncture needle 13 side, as shown in Figures 7 and 8.

Furthermore, a columnar gripping part 19 which acts as the retracting operating member of the present invention is mounted on the upper end part of the insertion rod 15a on the linear gripping member 15. Furthermore, an expandable engaging part 21 is attached to the portion in the vicinity of the gripping part 19 on the insertion rod 15a, and a coil spring 22 which covers the outer peripheral surface of the upper part portion of the insertion rod 15a is fixed to the lower end surface of the expandable engagement part 21. The lower end part of said coil spring 22 is supported by the bottom part of the gripping part 18.

The expandable engagement part 21 is configured by a flexible resin member, with a recess part 23a being formed on one side on the upper surface and also a recess part 23b being formed on the other side on the lower surface; the configuration is such that said expandable engagement part can expand or contract in the longitudinal direction (the left-right direction in Figures 3 to 5) by the application of a prescribed force. Furthermore, the lower surface of an end part 24 on one side of the expandable engagement part 21 is formed with an inclined surface 24a. Consequently, when the coil spring 22 is contracted by the downwards pressure (downwards movement of the insertion rod 15a) of the expandable engagement part 21 from the situation shown in Figure 3, the expandable engagement part 21 contracts and enters the penetration hole 18a of the gripping part 18 while the inclined surface 24a and the other end part are pushed against the inner peripheral surface of the penetration hole 18a, as shown in Figure 4.

Then, when the end part 24 of the expandable engagement part 21 is positioned in the engagement hole 18b, the expandable engagement part 21 expands and the end part 24 and the engagement hole 18b engage. The expandable engagement part 21 is fixed with respect to the gripping part 18 due to the engagement between said end part 24 and engagement hole 18b. When the end part 24 of said expandable engagement part 21 engages with the engagement hole 18b, a configuration is realized so that the gripping part 15b of the linear gripping member 15 protrudes outwards from the opening part 14b of the retrieval puncture needle 14 and extends to spread to the insertion puncture needle 13 side.

Moreover, the elastic member which acts as the advancement prevention means of the present invention is configured by the coil spring 22, and the advancement side fixing means of the present invention are configured by the expandable engagement part 21 and the engagement hole 18b of the gripping part 18. Furthermore, the retrieval puncture needle 14 is inserted and held in the holding hole 11b of the upper part holding tool 11 and the holding hole 12b of the lower part holding tool 12 in a state in which the opening part 14b is oriented towards the insertion puncture needle 13 side, and the upper part holding tool 11 and the lower part holding tool 12 hold the retrieval puncture needle 14 in a state in which it is parallel with the insertion puncture needle 13. Furthermore, the gripping parts 18, 19 are configured by an article moulded from resin material, and the suturing thread 16 is configured by a fine wire comprising resin material.

With this configuration and the use of the medical suturing tool 10, when the abdominal wall and the gastric wall of the patient are sutured, for example, the expandable engagement part 21 is first of all pulled upwards of the gripping part 18 by means of the elasticity of the coil spring 22, and a state is achieved in which the upper part portion of the insertion rod 15a is made to protrude upwards of the gripping part 18. By virtue of this, the linear gripping member 15 is pulled upwards and is positioned inside the insertion hole 14a of the retrieval puncture needle 14. In this state, the medical suturing tool 10 is pushed into the surface of the patient's skin in the abdomen part, and, as shown in Figure 6, the insertion puncture needle 13 and the retrieval puncture needle 14 pierce the abdominal wall A and the gastric wall B. It should be noted that Figures 6 to 9 which are used for the description given below are drawings showing the medical suturing tool 10 schematically, and the sizes etc. of all the elements are different from those of the medical suturing tool 10 shown in Figures 1 and 2.

In this case, the insertion puncture needle 13 and the retrieval puncture needle 14 are pushed in until the lower part holding tool 12 reaches the surface of the skin of the abdominal wall A, and the opening parts 13b, 14b are positioned inside the gastric wall B. Next, the gripping part 19 is pushed downwards to push the upper part portion of the insertion rod 15a inside the insertion hole 14a of the retrieval puncture needle 14. By virtue of this, the gripping part 15b of the linear gripping member 15 is sent outwards of the retrieval puncture needle 14 from the opening part 14b, it bends in a horizontal direction and also forks with a gap between the tip end parts, extending to the insertion puncture needle 13 side, and the situation shown in Figure 7 is achieved.

In this case, the end part 24 of the expandable engagement part 21 and the engagement hole part 18b of the gripping part 18 engage, whereby the expandable engagement part 21 is fixed with respect to the gripping part 18. Consequently, the linear gripping member 15 is also immobilized and the situation shown in Figure 7 is maintained. Next, as shown in Figures 1 and 8, the suturing thread 16 is inserted inside the insertion hole 13a of the insertion puncture needle 13 from the guide hole 17a of the gripping part 17. By virtue of this, the tip end part of the suturing thread 16 advances downwards inside the insertion hole 13a, and extends outside and downwards from the opening part 13b. At this time the tip end part of the suturing thread 16 enters between the fork of the extended gripping part 15b.

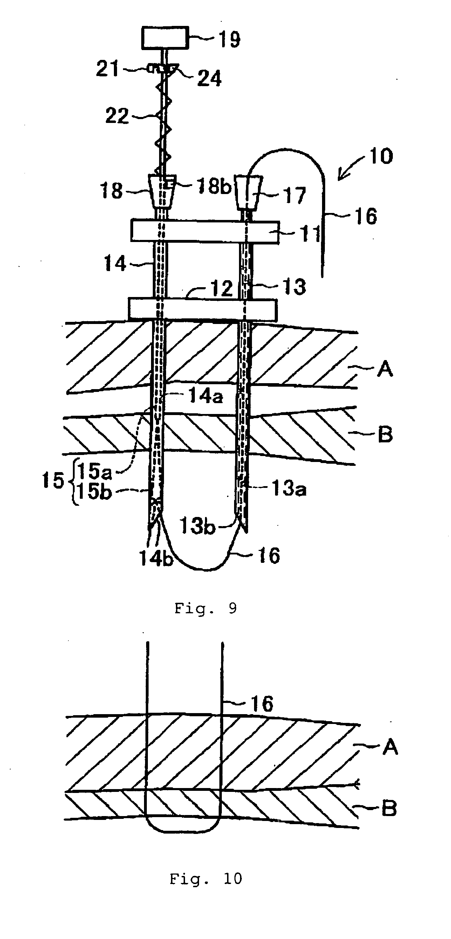

Next, the end part 24 of the expandable engagement part 21 is pushed inside the gripping part 18 and the engagement between the end part 24 and the engagement hole 18b is released. At this time, the upper part portion of the insertion rod 15a is raised so as to protrude upwards of the gripping part 18 due to the elasticity of the coil spring 22, and the insertion rod 15a together with the gripping part 15b are also pulled upwards to achieve the situation shown in Figures 2 and 9. At this time, the gripping part 15b of the linear gripping member 15 deforms so as to narrow the forked portion, keeping the suturing thread 16 within said forked portion, and the tip end part of the suturing thread 16 enters the insertion hole 14a of the retrieval puncture needle 14 in a state in which it is gripped by the gripping part 15b.

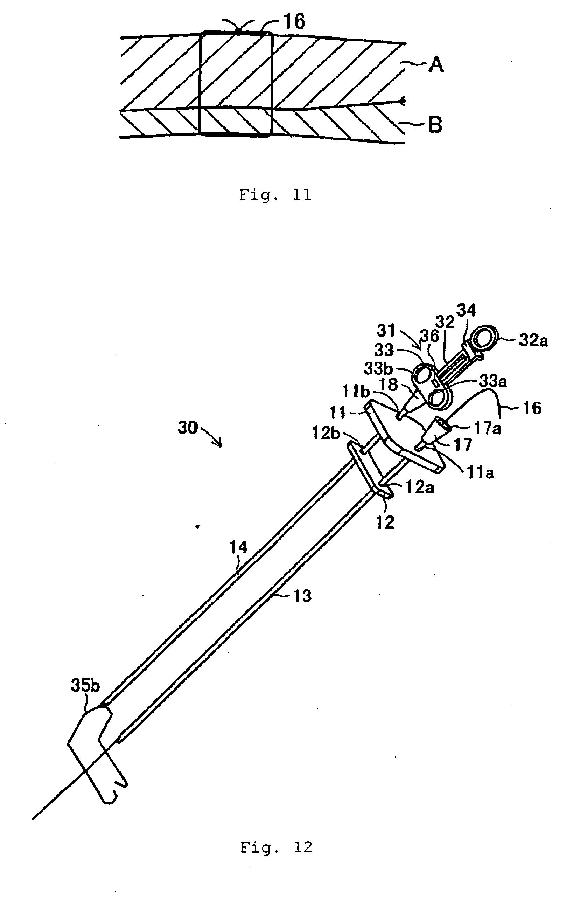

Accordingly, in the situation in Figure 9, a state is achieved in which the gastric wall B and the abdominal wall A are joined and both side portions of the suturing thread 16 extend outside the patient's body, this being due to the fact that the medical suturing tool 10 is pulled out of the patient's body. The situation in shown in Figure 10 is achieved by cutting so that both protruding side portions of said suturing thread 16 are a prescribed length. Then, both end parts of said suturing thread 16 are tied, whereby the situation of Figure 11 is reached and the suture is complete. It should be noted that the elastic force of the coil spring 22 is set to be greater than the force by which the linear gripping member 15 is pulled by the suturing thread 16 when the medical suturing tool 10 is pulled out from the body of the patient. Consequently, when the medical suturing tool 10 is pulled out from the body of the patient, the linear gripping member 15 is prevented from returning inside the insertion hole 14a of the retrieval puncture needle 14, and the suturing thread 16 is held in an engaged state with the gripping part 15b. As a result, stable suturing is possible.

As described above, with this medical suturing tool 10 the insertion rod 15a on the base end part side of the linear gripping member 15 is urged upwards by the coil spring 22. Consequently, when the suturing thread 16 is gripped by the gripping part 15b and is pulled inside the insertion hole 14a of the retrieval puncture needle 14, the insertion rod 15a is pulled inside the insertion hole 14a and it is possible to prevent the grip on the suturing thread 16 by the gripping part 15b from being released. By virtue of this, it is possible to pull the retrieval puncture needle 14 and the insertion puncture needle 13 out from the patient's body without manually handling the linear gripping member 15 or the suturing thread 16.

Furthermore, at that time the engagement between the gripping part 15b and the suturing thread 16 is not readily released, and therefore the handling stability is improved. In addition, the expandable engagement part 21 is provided on the insertion rod 15a and also the engagement hole 18b which can engage with the end part 24 of the expandable engagement part 21 is provided in the gripping part 18, and therefore it is possible to fix the position of the gripping part 15b when the gripping part 15b of the linear gripping member 15 is protruding from the opening part 14b of the retrieval puncture needle 14. Consequently, this simplifies handling when the tip end part of the suturing thread 16 is made to protrude from the opening part 13b of the insertion puncture needle 13 to engage with the gripping part 15b. In other words, there is no need to manually hold the gripping member 19 back when the suturing thread 16 is inserted inside the insertion puncture needle 13.

### Second Embodiment

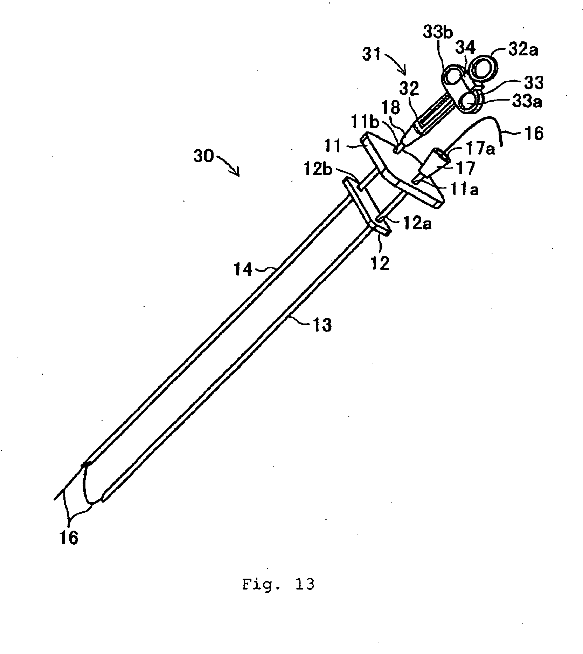

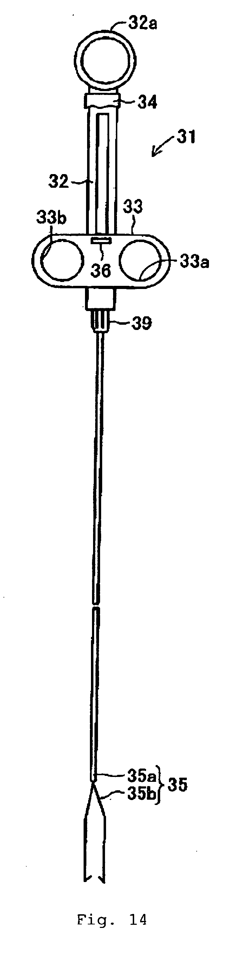

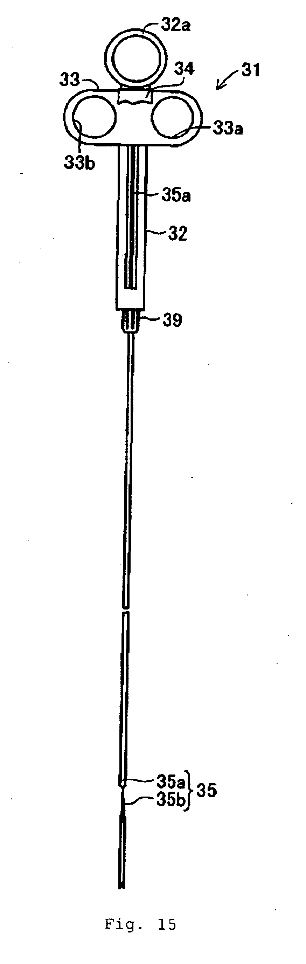

Figures 12 and 13 show a medical suturing tool 30 pertaining to the second mode of embodiment of the present invention. In this medical suturing tool 30 the retreat side fixing member, acting as a retracting operating member and advancement prevention means which are linked to the upper end part of a linear gripping member 35, is configured by a sliding engagement member 31, as shown in Figures 14 and 15. Said sliding engagement member 31 is configured by a longitudinal rail part 32 which extends vertically, a sliding part 33 which can slide vertically along the rail part 32, and an insertion tube 39 which is linked to the central lower end of the rail part 32.

The inside of the rail part 32 has the form of a frame which creates a space, and an annular gripping part 32a is provided at the top end part. Then, an engagement part 34 with a roughly L-shaped cross section which protrudes forwards and then extends curving downwards is formed at the upper end part of the rail part 32. It should be noted that an engagement protuberance is provided on the reverse side of a piece which extends downwards of the engagement part 34, although this is not shown in the figures. Furthermore, the sliding part 33 is configured by an elliptical plate body which is longer in the lateral direction, and gripping hole parts 33a, 33b are formed on both lateral sides thereof. Then, an engagement recess part 36 which can engage with (the engagement protuberance of) the engagement part 34 (this is referred to below simply as "the engagement part 34") is formed in the central upper part on the front surface of the sliding part 33.

Said engagement part 34 and engagement recess part 36 engage when the sliding part 33 is moved to the upper part of the rail part 32, and the engagement is released from this state when the sliding part 33 is moved to the lower part of the rail part 32. An insertion tube 39 is configured from a tube body made of resin and the linear gripping member 35 can be inserted therein. Then, the upper end part of an insertion rod 35a of the linear gripping member 35 is linked to the lower end central part of the sliding part 33. The linear gripping member 35 moves inside the retrieval puncture needle 14 in accordance with the vertical movement of the sliding part 33. Figures 14 and 15 show the state of such a linear gripping member 35.

To expedite the description, it should be noted that Figures 14 and 15 do not show the retrieval puncture needle 14, but, as shown in Figure 14, when the sliding part 33 is moved to the lower part of the rail part 32, the upper end part of the insertion rod 35a enters the retrieval puncture needle 14, and the gripping part 35b protrudes from the tip end part of the retrieval puncture needle 14. By virtue of this, the gripping part 35b spreads out. Furthermore, as shown in Figure 15, when the sliding part 33 is moved to the upper part of the rail part 32, the upper end side portion of the insertion rod 35a enters the inner side of the frame-shaped rail part 32, and most of the gripping member 35b retreats to enter the retrieval puncture needle 14. By virtue of this, the gripping part 35b extends in a straight line.

Moreover, the retracting operating member pertaining to the present invention is configured by the sliding part 33, and the retreat side fixing member which acts as the advancement prevention means pertaining to the present invention is configured by the engagement part 34 and the engagement recess part 36. Furthermore, the medical suturing tool 30 is not provided with advancement side fixing means. The configuration of the other components of said medical suturing tool 30 is the same as that of the medical suturing tool 10 described above. Accordingly identical components carry identical references, and a description thereof will be omitted.

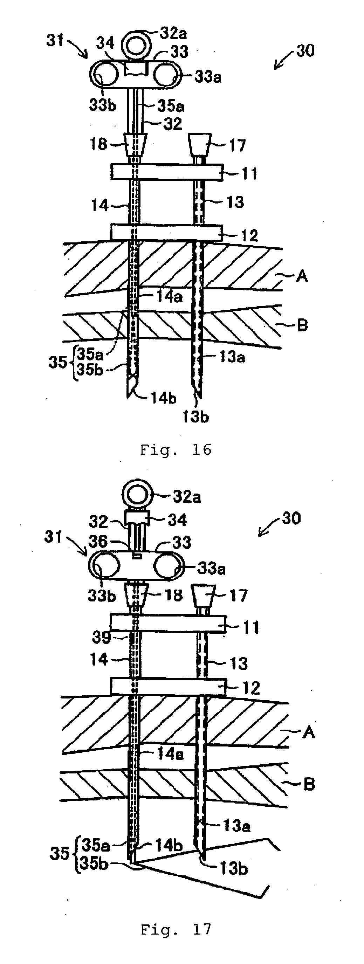

Using the medical suturing tool 30 configured in this way, when the abdominal wall A and the gastric wall B of the patient are sutured, the sliding part 33 is firstly pulled up to the upper part of the rail part 32, and the engagement part 34 and the engagement recess part 36 are set so as to be in an engaged state. By virtue of this, the linear gripping member 35 is pulled upwards, and the gripping part 35b is positioned inside the insertion hole 14a of the retrieval puncture needle 14. Figure 15 shows the linear gripping member 35 in this state. Next, in this state, the medical suturing tool 30 is pushed into the surface of the patient's skin in the abdomen part, and, as shown in Figure 16, the insertion puncture needle 13 and the retrieval puncture needle 14 pierce the abdominal wall A and the gastric wall B.

It should be noted that Figures 16 to 19 which are used for the description given below are drawings showing the medical suturing tool 30 schematically, and the sizes etc. of all the elements are different from those of the medical suturing tool 30 shown in Figures 12 and 13. Next, the sliding part 33 is moved downwards, and the insertion rod 35a of the linear gripping member 35 is pushed inside the insertion hole 14a of the retrieval puncture needle 14. By virtue of this, the gripping part 35b of the linear gripping member 35 is sent outwards of the retrieval puncture needle 14 from the opening part 14b, and the situation shown in Figure 17 is reached. Figure 14 shows the linear gripping member 35 in this state. It should be noted that in Figure 14 the sliding engagement member 31 is shown as seen from the front, and the linear gripping member 35 is shown seen from a direction that is different from that shown in Figure 17.

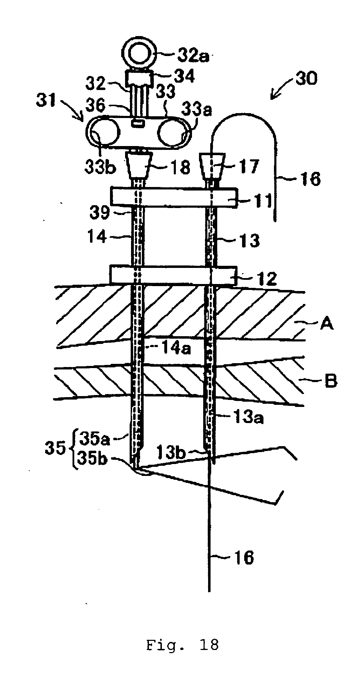

In this case, the sliding part 33 reaches a state of immobilization with respect to the rail part 32, due to friction force. Consequently, the linear gripping member 35 is also immobilized, and the situation shown in Figure 17 is maintained. Next, as shown in Figures 12 and 18, the suturing thread 16 is inserted inside the insertion hole 13a of the insertion puncture needle 13 from the guide hole 17a of the gripping part 17. By virtue of this, the tip end part of the suturing thread 16 extends downwards to the outside from the opening part 13b, and enters within the fork of the extended gripping part 35b.

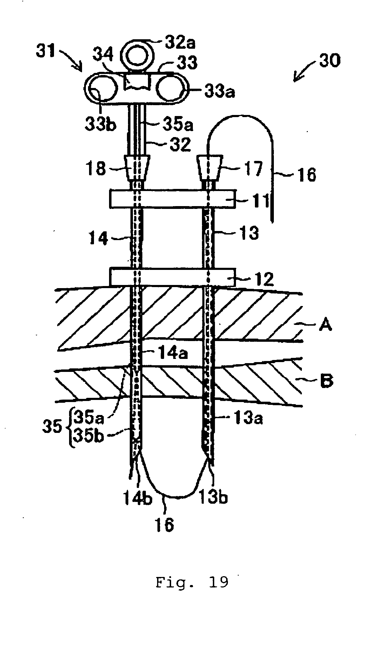

Next, the sliding part 33 is moved upwards, and the engagement part 34 and the engagement recess part 36 engage to reach the situation in Figure 19. At this time, the gripping part 35b of the linear gripping member 35 deforms so as to narrow the forked portion, keeping the suturing thread 16 within said forked portion, and the tip end part of the suturing thread 16 enters the insertion hole 14a of the retrieval puncture needle 14 in a state in which it is engaged with the gripping part 35b. In this case, the suturing thread 16 is fixed in a state in which it is pushed into the opening part 14b of the retrieval puncture needle 14 along with the tip end portion of the gripping part 35b. Accordingly, in the situation in Figure 19, a state is achieved in which the gastric wall B and the abdominal wall A are joined and both side portions of the suturing thread 16 extend outside the patient's body, this being due to the fact that the medical suturing tool 30 is pulled out of the patient's body.

The situation shown in Figure 10 is achieved by cutting so that both protruding side portions of said suturing thread 16 are a prescribed length, and in addition both end parts of said suturing thread 16 are tied, whereby the situation of Figure 11 is reached and the suture is complete. It should be noted that the engagement force of the engagement part 34 with the engagement recess part 36 is set to be greater than the force by which the linear gripping member 35 is pulled by the suturing thread 16 when the medical suturing tool 30 is pulled out from the body of the patient. Consequently, when the medical suturing tool 30 is pulled out from the body of the patient, the linear gripping member 35 is prevented from returning inside the insertion hole 14a of the retrieval puncture needle 14, and the suturing thread 16 is held in an engaged state with the gripping part 35b.

In this way, with this medical suturing tool 30 the advancement prevention means are configured by the engagement part 34 which is provided on the rail part 32 and the engagement recess part 36 which is provided on the sliding part 33, and therefore it is possible to reliably fix the sliding part 33 and the linear gripping member 35 by means of the engagement of the engagement part 34 with the engagement recess part 36. Furthermore, it is possible to allow the movement of the sliding part 33 and the linear gripping member 35 by releasing the engagement between the engagement part 34 and the engagement recess part 36. The other efficacious actions of this medical suturing tool 30 are the same as for the medical suturing tool 10 described above.

Furthermore, the medical suturing tool pertaining to the present invention is not limited to the modes of embodiment described above, and appropriate modifications may be implemented. For example, with the medical suturing tools 10, 30 described above, the gripping parts 15b, 35b are configured as forked linear bodies, but it is possible to use a configuration with a loop-shaped linear body as said gripping part. Furthermore, the structure of the insertion puncture needle 13 and the retrieval puncture needle 14 is not limited to the modes of embodiment described above either, and any kind of structure is possible provided that it is a structure which allows the engagement of the gripping part 15b with the suturing thread 16 outside the opening parts 13b, 14b. In addition, in the modes of embodiment described above the insertion puncture needle 13 and the retrieval puncture needle 14 are configured from stainless steel, but a resin material may also be used as the material for configuring them.

Furthermore, the upper part holding tool 11 and the lower part holding tool 12 may be configured by members which are long in the longitudinal direction, instead of being plate-shaped. By means of this, the upper part holding tool 11 and the lower part holding tool 12 are easier to hold manually, and they are easier to handle when a piercing is made. Furthermore, the insertion puncture needle 13 and the retrieval puncture needle 14 are more strongly fixed to the holding tools. By virtue of this, when a piercing is made, it is possible to prevent the tip end parts of the insertion puncture needle 13 and the retrieval puncture needle 14 turning or bending so as to extend with a gap between them. In addition, it is possible to configure the upper part holding tool 11 and the lower part holding tool 12 as large components, and to provide a plurality of insertion puncture needles 13 and retrieval puncture needles 14. By virtue of this it is possible to carry out several suturing operations in a single turn, thereby reducing the time taken for the operation and improving efficiency. In addition, the medical suturing tools 10, 30 pertaining to the present invention are not limited to suturing the abdominal wall A and the gastric wall B, and they may be used for suturing at other locations in the body.

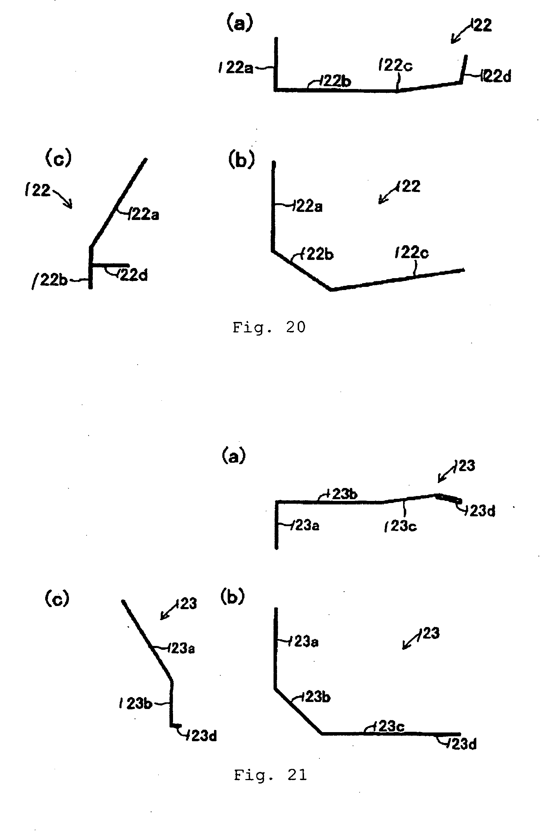

Gripping part 15b may be formed in the shapes shown in Figs 20 and 21. As shown, the gripping part 15b comprises linear coupling portions 122 and 123. Figures 20 and 21 illustrate linear coupling portions 122 and 123 in their natural state without deformation due to the application of a force. Figures 20(a) and 21(a) are plan views; Figures 20(b) and 21(b) are front views; and Figures 20(c) and 21(c) are side views. As shown in Figure 20, linear coupling portion 122 is composed of hanging-down portion 122a that extends from the lower end of base portion 15a (not shown in the figure) obliquely downward (in a direction away from linear coupling portion 123), inclined portion 122b that extends from the lower end of hanging-down portion 122a obliquely downward (in the direction parallel to linear coupling portion 123), and nearly horizontal portion 122c that is bent from the tip portion of inclined portion 122b and extends slightly to the upper side (parallel to linear coupling portion 123 in the plan view).

On the tip of nearly horizontal portion 122c, bending portion 122d extending nearly parallel to hanging-down portion 122a in the plan view and toward linear coupling portion 123 is formed. Also, as shown in Figure 21, linear coupling portion 123 is composed of hanging-down portion 123a that extends from the lower end (not shown in the figure) of base portion 15a obliquely downward (in a direction away from linear coupling portion 122), inclined portion 123b that is bent from the lower end of hanging-down portion 123a and extends obliquely downward parallel to inclined portion 122b, and nearly horizontal portion 123c that is bent from the tip portion of inclined portion 123b and extends in a nearly horizontal direction parallel to nearly horizontal portion 122c. On the tip of nearly horizontal portion 123c, bending portion 123d formed in a slender U-shape in the plan view is formed.

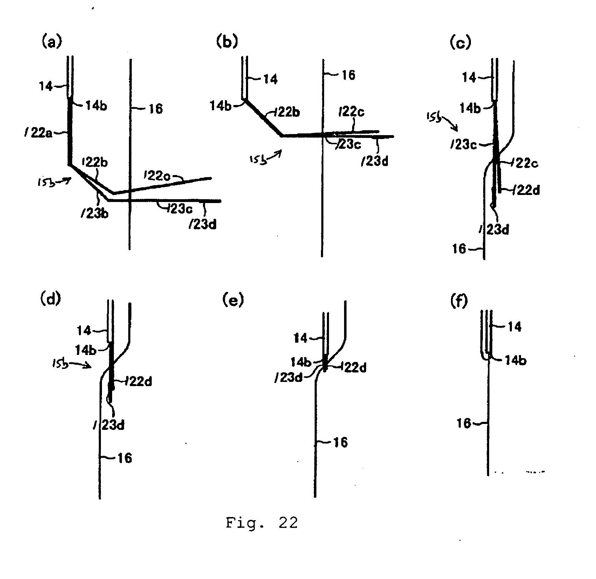

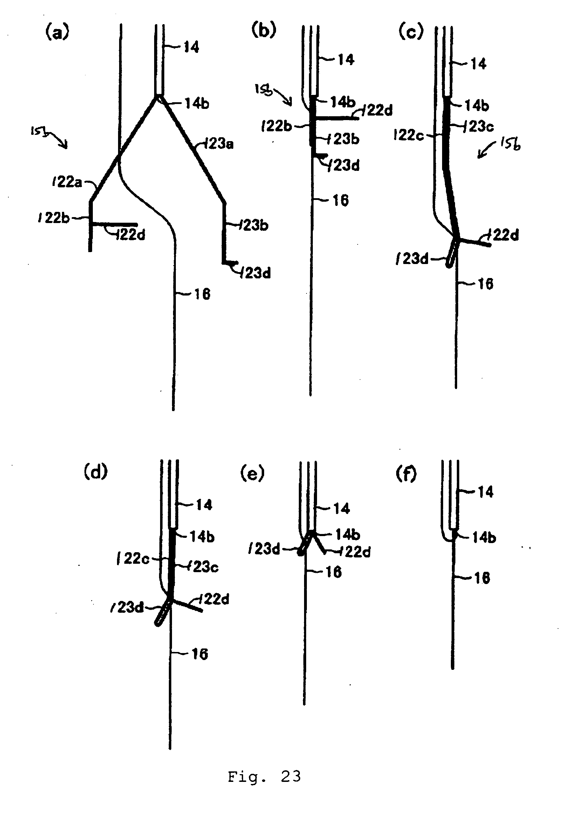

The operational relationship between gripping portion 15b and a suture 16 changes as shown in Figures 22 and 23. As shown in Figures 22 and 23, gripping portion 15b and suture 16 are coupled to each other and enter the interior of insertion hole 14a of puncture needle 14. Figure 22 is a diagram illustrating the state as seen from the front, and Figure 23 shows the state as seen from the side. Figures 22(a) and 23(a) illustrate gripping portion 15b and suture 16 in the same state as that shown in Figure 8. Then, from the state shown in Figures 22(a) and 23(a), linear gripping member 15 is pulled up slightly, and hanging-down portions 122a, 123a approach each other to become linear, and they enter puncture needle 14. As a result, as shown in Figures 22(b) and 23(b), inclined portions 122b, 123b also approach each other and tightly contact each other, and suture 16 is held between nearly horizontal portions 122c, 123c.

Then, linear gripping member 15 is pulled up slightly, and inclined portions 122b, 123b enter puncture needle 14. As shown in Figures 22(c) and 23(c), nearly horizontal portions 122c, 123c cross each other and are intertwined, so that suture 16 is fastened. Then, linear gripping member 15 is pulled up slightly, and the upper portions of nearly horizontal portions 122c, 123c enter puncture needle 14. As a result, as shown in Figures 22(d) and 23(d), since nearly horizontal portions 122c and 123c are in tight contact with each other, suture 16 is held by them. Then, linear gripping member 15 is pulled up and the remaining portions of nearly horizontal portions 122c, 123c enter puncture needle 14, so that suture 16 hits the edge portion of opening portion 14b and is positioned on the lower side between nearly horizontal portions 122c, 123c.

In this state, said bending portions 122d, 123d are intertwined and are coupled to each other, and, as shown in Figures 22(e) and 23(e), suture 16 is coupled to the coupling portion of bending portions 122d, 123d. As a result, when linear gripping member 15 is further pulled into puncture needle 14, as shown in Figures 22(f) and 23(f), the entirety of gripping portion 15b enters puncture needle 14, and the portion of coupling between suture 16 and gripping portion 15b goes together with gripping portion 15b into puncture needle 14. Said Figures 22(f) and 23(f) correspond to Figures 2 and 9, respectively. Consequently, in the state shown in Figures 2 and 9, when suturing unit for medical use 10 is pulled out of the body of the patient, the two side portions of suture 16 extend out of the body of the patient such that stomach wall B is bonded to stomach wall A.

### Third Embodiment

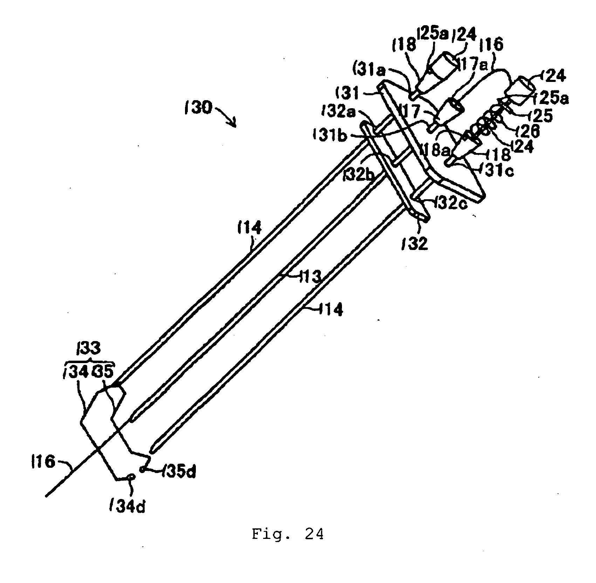

Figure 24 is a diagram illustrating suturing unit for medical use 130 of a third embodiment of the present invention. Said suturing unit for medical use 1301 has one insertion puncture needle 113 of the same structure as that of said puncture needle 13, and two retrieval piercing needles 114 having the same structure as that of said puncture needle 14. Also, upper holding unit 131 and lower holding unit 132 that hold said one insertion puncture needle 113 and two retrieval puncture needles 114 are formed in a rectangular shape with the major side in the left/right direction with respect to said upper holding unit 111 and lower holding unit 112.

On said upper holding unit 131, three round holding holes 131a, 131b, 131c with a prescribed spacing between them are formed, and, on lower holding unit 132, round holding holes 132a, 132b, 132c are formed with the same spacing as that for said round holding holes 131a, 131b, 131c. Also, one of two retrieval puncture needles 14 is held to upper holding unit 131 and lower holding unit 132 via round holding holes 131a and 132a, insertion puncture needle 13 is held via holding hole 131b and holding hole 132b in upper holding unit 131 and lower holding unit 132. The orientation and position relationship between said one puncture needle 14 and insertion puncture needle 13 is the same as that in said Embodiment 1.

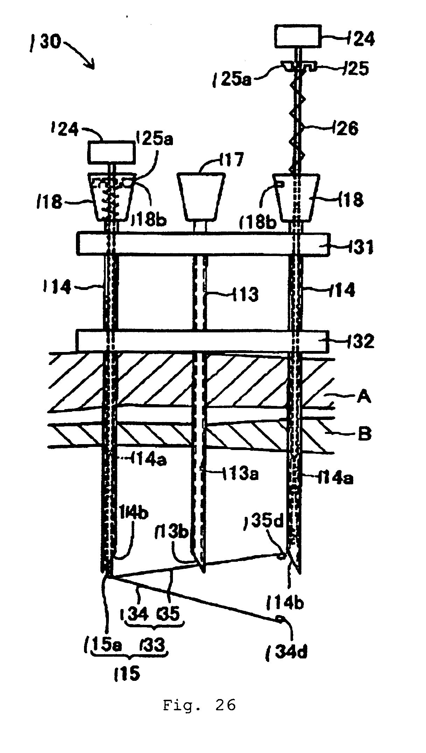

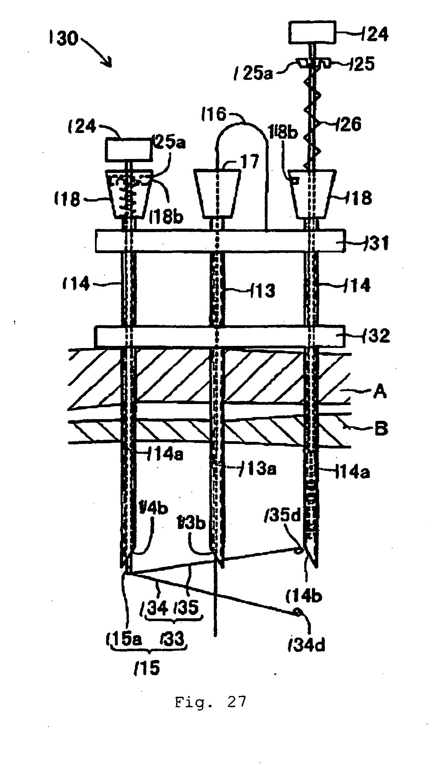

The other retrieval puncture needle 114 is held via holding hole 131c and holding hole 132c in said upper holding unit 131 and lower holding unit 132 having opening portion 114b facing opening portion 114b of one of the other retrieval puncture needle 114. Also, bending portions 134d, 135d of linear coupling portions 134, 135 that form gripping portion 133 are formed bent round to form a loop. The constitution of the remaining portion of said suturing unit for medical use 130 is the same as that of said suturing unit for medical use 10. Consequently, the same parts as those adopted in the above are adopted, and they will not be explained again. Also, the shape of the portion of holding portion 133 other than bending portions 134d, 135d is the same as that of said gripping portion 15b. However, the structure is shown only schematically in Figure 24 and Figures 26, 27, 29 and 30 to be used in the following explanation.

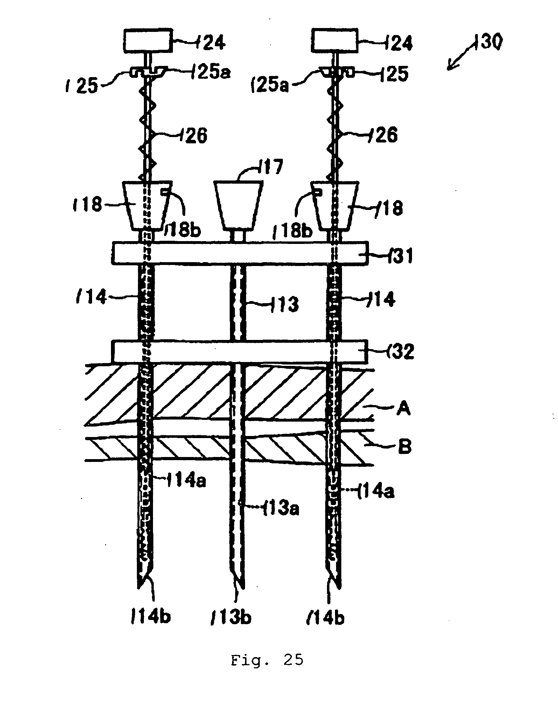

When suturing unit for medical use 130 with said constitution is used to perform suturing for abdominal wall A and stomach wall B of a patient, the suturing operation is performed as shown in Figures 25-33. Figures 25-31 to be used in the following explanation are schematic diagrams illustrating suturing unit for medical use 130. The sizes and shapes of the various portions are different from those of suturing unit for medical use 130 shown in Figure 24. In this case, first of all, due to the elasticity of coil spring 126, stretching coupling portion 125 is pushed up above holding portion 118, and upper portions of base portions 15a of linear gripping members 15 attached on the two retrieval puncture needles 14 protrude above holding portion 118.

Here, suturing unit for medical use 130 in this state is pressed into the surface of the skin in the abdominal portion of the patient, and, as shown in Figure 25, puncture needle 13 and two puncture needles 14 are pierced into abdominal wall A and stomach wall B. Also, the operation in the state shown in Figures 25-38 is the same as that shown in Figures 6-9. During this operation, holding portion 133 and suture 116 are coupled to each other while the state is changed as shown in Figures 22 and 23.

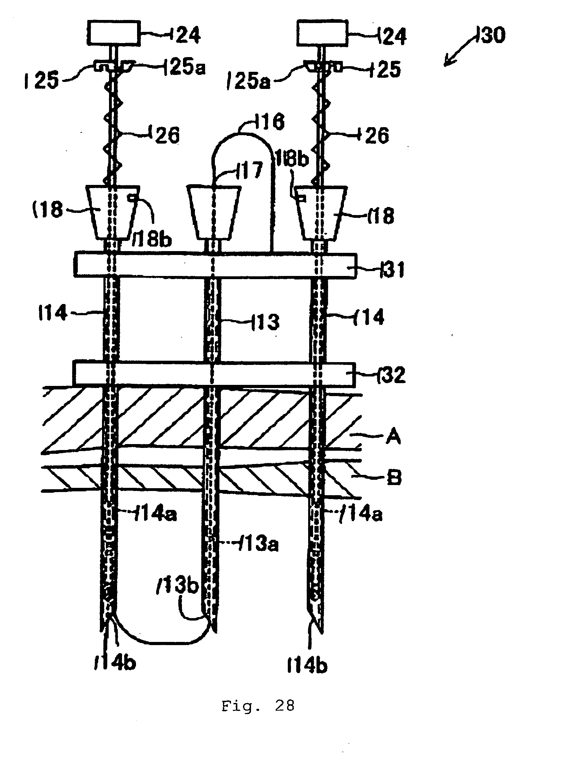

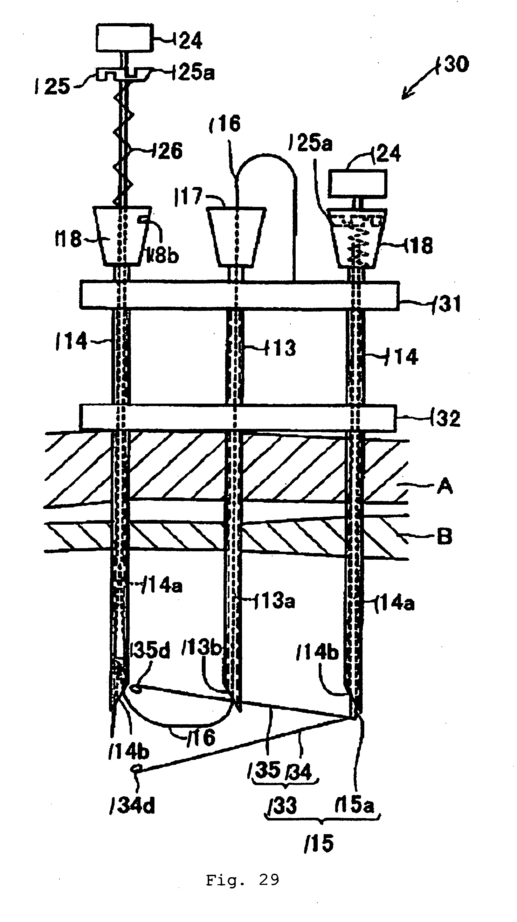

As shown in Figure 28, the tip portion of suture 116 inserted from puncture needle 113 is coupled to gripping portion 133 of linear gripping member 115 inserted in one of puncture needles 114, and it enters puncture needle 114. From this state, gripping portion 124 of the other puncture needle 114 is pressed down, so that the upper portion of base gripping portion 115a is pressed into inserting hole 114a of puncture needle 114. As a result, gripping portion 133 exits opening portion 114b to the exterior of puncture needle 114, and, while it is bent in the horizontal direction, it stretches to the sides of puncture needle 113 so that the spacing between linear coupling portions 134, 135 spreads, and the state is as shown in Figure 29.

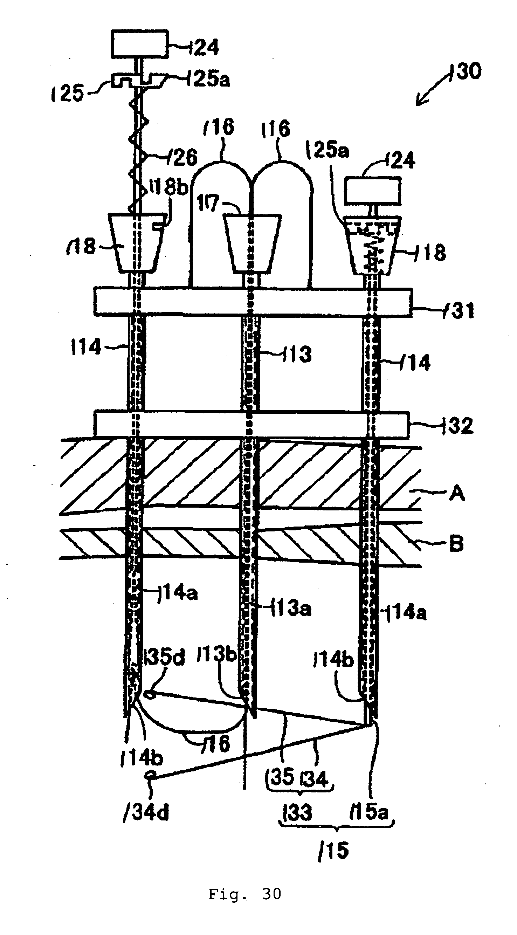

In this case, end portion 125a of stretching coupling portion 125 and coupling hole 118b of holding portion 118 are coupled to each other, and stretching coupling portion 125 is fixed with respect to holding portion 118. As a result, linear gripping member 115 is also in a stationary state, and the state shown in Figure 29 is maintained. Then, as shown in Figure 30, suture 116 is inserted in inserting hole 113a of puncture needle for insertion 113, and its tip portion protrudes below and outside opening portion 113b. As a result, the tip portion of suture 116 enters between linear coupling portions 134, 135 of gripping portion 133 that has been expanded.

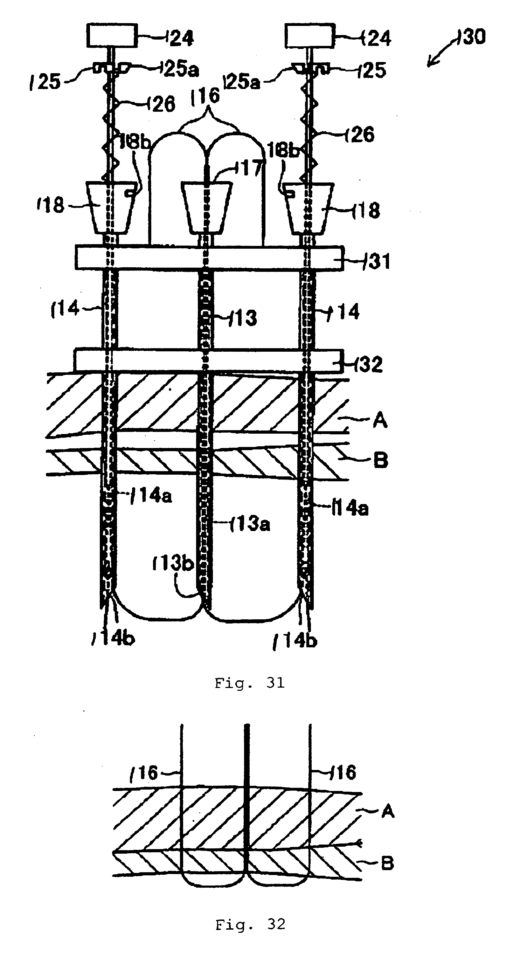

Then, end portion 125a of stretching coupling portion 125 is forced into holding portion 118, and coupling between end portion 125a and coupling hole 118b is released. Due to the elasticity of coil spring 126, the upper portion of base portion 115a is protruded to above holding portion 118. As a result, together with base portion 115a, gripping portion 133 is also pulled up to the state shown in Figure 31. In this case, for gripping portion 133, the spacing between linear coupling portions 134, 135 decreases, and deformation takes place such that they are intertwined to hold suture 116. The tip portion of suture 116 is fixed in opening portion 114b of puncture needle 114 while held by gripping portion 133, or it may enter inserting hole 114a and be fixed in inserting hole 114a.

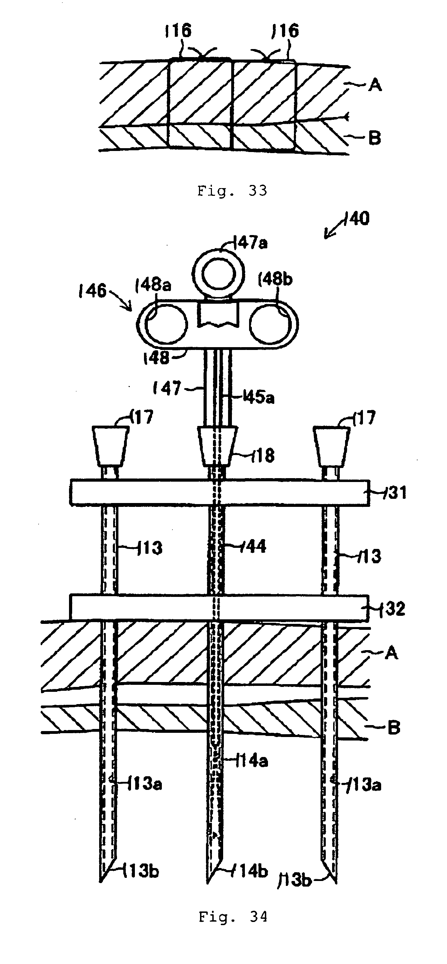

Consequently, in the state shown in Figure 31, by pulling suturing unit for medical use 130 out of the body of the patient, two sutures 116 extend out of the body of the patient for two side portions such that stomach wall B and abdominal wall A are bonded to each other. Then, by cutting the two protruding side portions of said two sutures 116 to a prescribed length, the state becomes as shown in Figure 32. In addition, by tying the two end portions of sutures 116, respectively, suturing comes to an end in the state shown in Figure 33.

As a result, in a single round of operation, two sutures 116 can be used in suturing. Consequently, the suturing operation is simplified, and the operation time is decreased. Especially, when the suturing portion is large, the operation can be performed at high efficiency. Also, because bending portions 134d, 135d of linear coupling portions 134, 135 are formed to be round, stomach wall B is not harmed. The other functions of this suturing unit for medical use 130 are the same as those of said suturing unit for medical use 10.

### Fourth Embodiment

Figures 34-37 illustrate the state of the suturing operation using suturing unit for medical use 140 in Embodiment 4 of the present invention. Said suturing unit for medical use 140 has two insertion puncture needles 113 having the same structure as that of the puncture needle 113 of the third embodiment, and one retrieval puncture needle 144. Also, as the upper holding unit and lower holding unit for holding said two puncture needles 113 and one puncture needle 144, the same structures as those of upper holding unit 131 and lower holding unit 132 of Embodiment 3 are used. At the central portion between upper holding unit 131 and lower holding unit 132, puncture needle 144 is attached, and, on the two side portions of upper holding unit 131 and lower holding unit 132, puncture needles 113 are attached with a prescribed spacing from puncture needle 144.

Also, linear coupling portions 142, 143 that form holding portion 141 of linear holding member 145 are formed such that after spreading to an obtuse angle, bending occurs such that their tips approach each other, and bending portions 142d, 143d of the tip portions are made of bent pieces such that they face the base end portions of linear coupling portions 142, 143. Also, for suturing unit for medical use 140, instead of holding portion 124, slide engagement member 146 is used, similar to slide engagement member 31. This slide engagement member 146 is composed of longitudinal rail portion 147 extending in the upper/lower direction, and slide portion 148 that can slide up/down along rail portion 147.

Said rail portion 147 is formed in a frame shape with a space formed in its interior, and, on its upper end portion, ring-shaped holding portion 147a is set. Also, slide portion 148 is formed as an elliptic plate member that has its major axis in the left/right direction, with hole portions 148a, 148b for holding formed on its left/right sides. Although not shown in the figure, a coupling portion is formed on the upper end portion of rail portion 147, and at the center of the upper portion of slide portion 148, a coupled portion that can be coupled to the coupling portion of rail portion 147 is formed. This coupling portion and coupled portion are coupled to each other when slide portion 148 is driven to move to the upper end of rail portion 147. From this state, the coupling is released when slide portion 148 moves to the lower end of rail portion 147.

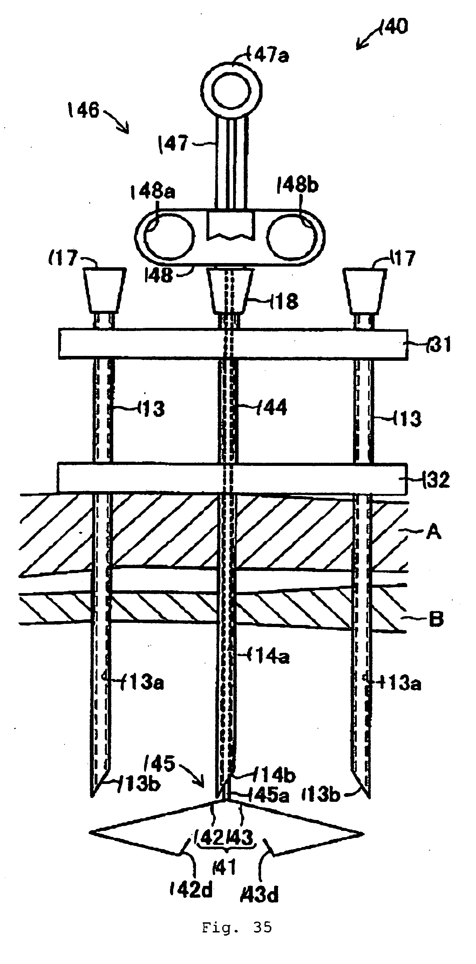

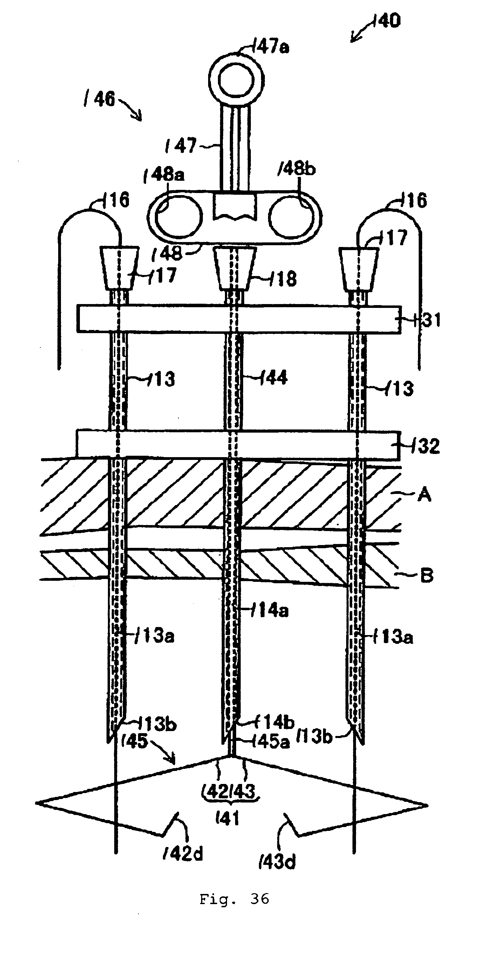

The upper end portion of base portion 145a of linear gripping member 145 is connected to the central portion of the lower end of slide portion 148, and, when said slide portion 148 moves up/down, linear gripping member 145 moves inside puncture needle 144. That is, as shown in Figures 35 and 36, when slide portion 148 moves to the lower end of rail portion 147, the upper portion of base portion 145a of linear gripping member 145 enters puncture needle 144, and gripping portion 141 protrudes and spreads from the tip opening of puncture needle 144.

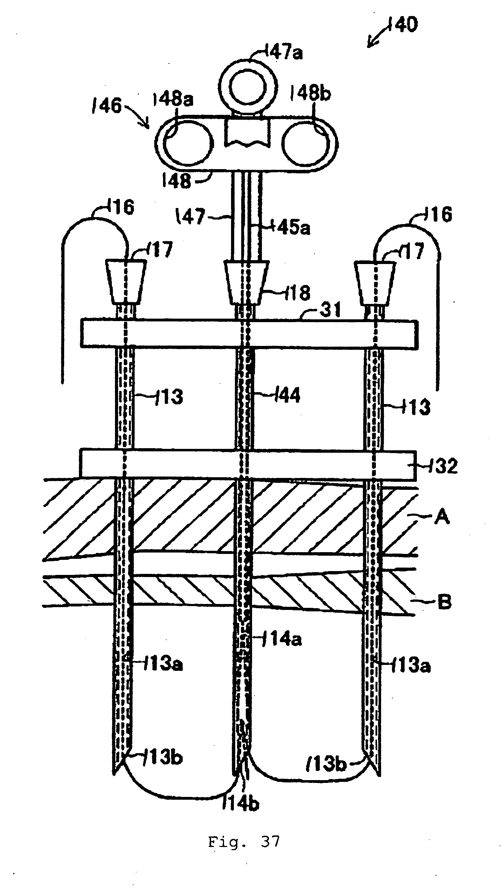

Also, as shown in Figures 34 and 37, when slide portion 148 is driven to move to the upper end of rail portion 147, the upper end portion of base portion 145a of linear gripping member 145 enters the inner side in the frame shape of rail portion 147, and gripping portion 141 retreats and enters puncture needle 144. The constitution of the remaining portion of suturing unit for medical use 140 is the same as that of said suturing unit for medical use 10. As a result, they will not be explained again.

When suturing unit for medical use 140 with said constitution is used to suture abdominal wall A and stomach wall B of the patient, first of all, slide portion 148 is pulled to the upper end of rail portion 147, and the coupling portion and the coupled portion are coupled to each other. As a result, linear gripping member 145 is pulled upward, and gripping portion 141 is positioned in puncture needle 144. Then, suturing unit for medical use 140 in this state is pressed into the skin surface of the abdominal portion of the patient, and, as shown in Figure 34, two puncture needles 113 and one puncture needle 144 pierce abdominal wall A and stomach wall B. Then, when slide portion 148 is driven to move downward, base portion 145a of linear gripping member 145 is pressed into puncture needle 144. As a result, gripping portion 141 of linear gripping member 145 exits puncture needle 144 from opening portion 114b to obtain the state shown in Figure 35.

In this case, due to frictional force, slide portion 148 becomes stationary with respect to rail portion 147. As a result, linear gripping member 145 is also stationary, and the state shown in Figure 35 is maintained. Then, as shown in Figure 36, sutures 116 are inserted into puncture needles 113 from guide holes 117a of holding portions 117 of two puncture needles 113. As a result, the tip portions of two sutures 116 extend below and outside opening portions 113b, and they enter expanded gripping portion 141. Then, slide portion 148 is driven to move upward, and the coupling portion and coupled portion are coupled to each other to obtain the state shown in Figure 37.

In this case, gripping portion 141 of linear gripping member 145 is deformed such that the spacing between linear coupling portions 142, 143 decreases to hold both sutures 116. The tip portions of said both sutures 116 enter puncture needle 144 while they are coupled to gripping portion 141. In this case, both sutures 116 are coupled to bending portions 142d, 143d of linear coupling portions 142, 143 and are fixed in the inserting tube. Consequently, in the state shown in Figure 37, by pulling suturing unit for medical use 140 from the body of the patient, both sutures 116 extend out from the patient for both side portions such that stomach wall B is bonded to stomach wall A.

The two protruding side portions of said two sutures 116 are cut at a prescribed length, and the state becomes the same as in Figure 32. In addition, by tying the two end portions of two sutures 116, the state becomes as shown in Figure 33, and suturing comes to an end. By means of said suturing unit for medical use 140, suturing can be performed for two sutures 116 simultaneously. As a result, the suturing operation can be performed in a simple way, and, at the same time, the operation time can be further shortened. The remaining functions of suturing unit for medical use 140 are the same as those of said suturing unit for medical use 10.

## Claims

1. A medical suturing tool comprising:
a plurality of suture insertion puncture needles (113);
a suture retrieval puncture needle (144) and
a frame (131, 132) for holding said plurality of suture insertion puncture needles and said a suture retrieval puncture needle in a spaced apart relationship,
wherein said suture retrieval puncture needle (144) comprises an outer hollow needle member (114a) and an inner suture gripping element (141) movable within said outer hollow needle member, said inner suture gripping element being deployable at least partially externally to said outer hollow needle member for retrieval of a suture inserted into at least one of said suture insertion puncture needles (113),
wherein an operating member (124, 146) is provided at a proximal end of said suture retrieval puncture needle, said operating member being connected to said inner suture gripping element, and wherein movement of said operating member relative to said outer hollow needle member causes said inner suture gripping element to move between a deployed position and a retracted position, and wherein the suture retrieval puncture needle comprises a coil spring (126) which covers the outer peripheral surface of the upper part portion of an insertion rod **characterized in that** said suture retrieval puncture needle further comprising a locking arrangement (125, 118b) for releasably holding said operating member in a position relative to said outer hollow needle member in which said inner suture gripping element is in said deployed position.

2. The medical suturing tool according to Claim 1, wherein said locking arrangement comprises a biasing element (126) for urging said operating member toward a position relative to said outer hollow needle member in which said inner suture gripping element is in said retracted position.

3. The medical suturing tool according to claim 2, wherein said biasing element (126) is a coil spring.

4. The medical suturing tool according to claim 1, wherein said locking arrangement comprises a cup-shaped body (118) attached to said outer hollow needle member and a deformable engaging element (125) attached to said operating member, said engaging element being arranged to releasable engage a recess (118b) in said cup-shaped body.

5. The medical suturing tool according to claim 1, wherein said operating member comprises a finger-operable slide member (146) arranged to be slidably movable within a housing element (148) attached to a proximal end of said outer hollow needle member.

6. The medical suturing tool according to claim 5, wherein said locking arrangement comprises a recess (36) in one of said slide member and said housing element and an engaging element on the other of said slide member and said housing element, arranged to releasable engage with said recess.

## Patentansprüche

1. Medizinisches Nahtinstrument, umfassend:
mehrere Nahteinführungspunktionsnadeln (113); eine Nahtrückholpunktionsnadel (144) und
einen Rahmen (131, 132) zum Halten der mehreren Nahteinführungspunktionsnadeln und der Nahtrückholpunktionsnadel in einer beabstandeten Beziehung,
wobei die Nahtrückholpunktionsnadel (144) ein äußeres hohles Nadelglied (114a) und ein inneres Nahtgreifelement (141), das in dem äußeren hohlen Nadelglied beweglich ist, umfasst, wobei das innere Nahtgreifelement zumindest teilweise außerhalb des äußeren hohlen Nadelglieds ausbringbar ist, um eine in mindestens eine der Nahteinführungspunktionsnadeln (113) eingesetzte Naht zurückzuholen,
wobei ein Betätigungsglied (124, 146) an einem proximalen Ende der Nahtrückholpunktionsnadel vorgesehen ist, wobei das Betätigungsglied mit dem inneren Nahtgreifelement verbunden ist und wobei Bewegung des Betätigungsglieds bezüglich des äußeren hohlen Nadelglieds bewirkt, dass sich das innere Nahtgreifelement zwischen einer ausgebrachten Position und einer zurückgezogenen Position bewegt, und wobei die Nahtrückholpunktionsnadel eine Schraubenfeder (126) umfasst, die die Außenumfangsfläche des oberen Teilabschnitts einer Einführstange bedeckt, **dadurch gekennzeichnet, dass** die Nahtrückholpunktionsnadel weiterhin eine Verriegelungsanordnung (125, 118b) zum lösbaren Halten des Betätigungsglieds in einer Position bezüglich des äußeren hohlen Nadelglieds umfasst, in der sich das innere Nahtgreifelement in der ausgebrachten Position befindet.

2. Medizinisches Nahtinstrument nach Anspruch 1, wobei die Verriegelungsanordnung ein Vorspannelement (126) zum Drücken des Betätigungsglieds in eine Position bezüglich des äußeren hohlen Nadelglieds umfasst, in der sich das innere Nahtgreifelement in der zurückgezogenen Position befindet.

3. Medizinisches Nahtinstrument nach Anspruch 2, wobei das Vorspannelement (126) eine Schraubenfeder ist.

4. Medizinisches Nahtinstrument nach Anspruch 1, wobei die Verriegelungsanordnung einen becherförmigen Körper (118), der an dem äußeren hohlen Nadelglied befestigt ist, und ein verformbares Eingriffselement (125), das an dem Betätigungsglied befestigt ist, umfasst, wobei das Eingriffselement zur lösbaren Ineingriffnahme einer Aussparung (118b) in dem becherförmigen Körper angeordnet ist.

5. Medizinisches Nahtinstrument nach Anspruch 1, wobei das Betätigungsglied ein fingerbetätigbares Gleitglied (146) umfasst, das zur Gleitbewegung in einem an einem proximalen Ende des äußeren hohlen Nadelglieds befestigten Gehäuseelement (148) angeordnet ist.

6. Medizinisches Nahtinstrument nach Anspruch 5, wobei die Verriegelungsanordnung eine Aussparung (36) in dem Gleitglied oder dem Gehäuseelement und ein Eingriffsglied an dem jeweils anderen, dem Gehäuseelement oder dem Gleitglied, umfasst, das zur lösbaren Ineingriffnahme der Aussparung angeordnet ist.

## Revendications

1. Outil de suture chirurgicale comprenant:
une pluralité d'aiguilles de ponction d'insertion de suture (113) ;
une aiguille de ponction de retrait de suture (144) et
un cadre (131, 132) pour retenir ladite pluralité d'aiguilles de ponction d'insertion de suture et
ladite aiguille de ponction de retrait de suture en relation espacée,
ladite aiguille de ponction de retrait de suture (144) comprenant un organe d'aiguille creux externe (114a) et un élément de préhension de suture interne (141) déplaçable dans ledit organe d'aiguille creux externe, ledit élément de préhension de suture interne étant déployable au moins en partie extérieurement vers ledit organe d'aiguille creux externe pour retirer une suture insérée dans au moins l'une desdites aiguilles de ponction d'insertion de suture (113),
un organe fonctionnel (124, 146) étant prévu à une extrémité proximale de ladite aiguille de ponction de retrait de suture, ledit organe fonctionnel étant connecté audit élément de préhension de suture interne, et le mouvement dudit organe fonctionnel par rapport audit organe d'aiguille creux externe amenant ledit élément de préhension de suture interne à se déplacer entre une position déployée et une position rétractée, et l'aiguille de ponction de retrait de suture comprenant un ressort à boudin (126) qui recouvre la surface périphérique externe de la portion de partie supérieure d'une tige d'insertion, **caractérisé en ce que** ladite aiguille de ponction de retrait de suture comprend en outre un agencement de verrouillage (125, 118b) pour retenir de manière amovible ledit organe fonctionnel dans une position par rapport audit organe d'aiguille creux externe dans laquelle ledit élément de préhension de suture interne est dans ladite position déployée.

2. Outil de suture chirurgicale selon la revendication 1, dans lequel ledit agencement de verrouillage comprend un élément de poussée (126) pour pousser ledit organe fonctionnel vers une position par rapport audit organe d'aiguille creux externe dans laquelle ledit élément de préhension de suture interne est dans ladite position rétractée.

3. Outil de suture chirurgicale selon la revendication 2, dans lequel ledit élément de poussée (126) est un ressort à boudin.

4. Outil de suture chirurgicale selon la revendication 1, dans lequel ledit agencement de verrouillage comprend un corps (118) en forme de coupelle attaché audit organe d'aiguille creux externe et un élément d'engagement déformable (125) attaché audit organe fonctionnel, ledit élément d'engagement étant agencé de manière à s'engager de manière amovible avec un retrait (118b) dans ledit corps en forme de coupelle.

5. Outil de suture chirurgicale selon la revendication 1, dans lequel ledit organe fonctionnel comprend un organe coulissant (146) actionnable au doigt agencé de manière à pouvoir se déplacer par coulissement dans un élément de boîtier (148) attaché à une extrémité proximale dudit organe d'aiguille creux externe.

6. Outil de suture chirurgicale selon la revendication 5, dans lequel ledit agencement de verrouillage comprend un retrait (36) dans l'un dudit organe coulissant et dudit élément de boîtier et un élément d'engagement sur l'autre dudit organe coulissant et dudit élément de boîtier, agencé de manière à s'engager de manière amovible avec ledit retrait.
